# EUROPEAN PATENT APPLICATION

(11) **EP 4 640 164 A1**
(43) Date of publication of application: **29.10.2025**
(21) Application number: 23905963.7
(22) Date of filing: 19.12.2023
(51) Int. Cl.: A61B 17/04

(54) **ELECTRIC SUTURING DEVICE**

(30) Priority: 22.12.2022 CN 202211652020
(71) Applicant: B. J. Zh. F. Panther Medical Equipment Co., Ltd., Beijing 102200 (CN)
(72) Inventor: FAN, Hongli, Beijing 102200 (CN)
(74) Representative: Huang, Liwei
(86) International application number: PCT/CN2023/139974
(87) International publication number: WO 2024/131800

(57) **Abstract**

The present invention discloses an electric suturing device, comprising: a working-end assembly, a main drive mechanism, a bending drive mechanism and a rotating drive mechanism; the working-end assembly comprising a suturing assembly and a bending connecting tube, wherein the main drive mechanism is used to drive a needle-and-thread component to reciprocally rotate to suture the to-be-sutured tissue, the bending drive mechanism is used to drive the working-end assembly to swing left and right in order to adjust the position of the working-end assembly relative to the to-be-sutured tissue, the rotating drive mechanism is used to drive the suturing assembly of the working-end assembly to achieve 360-degree rotation around the axis of the working end, so as to adjust the position of the needle-and-thread component relative to the entry point of the to-be-sutured tissue. By providing the main drive mechanism, the bending drive mechanism and the rotating drive mechanism, the present invention can facilitate the doctor to adjust the position of the suture component and perform the suture operation, so that the efficiency of the suture can be improved and the operation time can be shortened.

## Description

### Technical Field

The present invention relates to the field of medical devices, and in particular to an electrically operated suturing device.

### Background technology

Laparoscopic surgery is a newly developed minimally invasive method, which is an inevitable trend in the development of future surgical methods. With the rapid advancement of industrial manufacturing technology, the integration of related disciplines has laid a firm foundation for carrying out new technologies and methods, which, together with the increasingly skillful operation of the surgeon, has led to the fact that many of the open surgeries of the past have been replaced by endoluminal surgeries, which has greatly increased the chances of surgical choices. The traditional method of laparoscopic surgery is to first insert a tube-like working channel called "trocar", and then all operations are carried out through this tube; special lengthened surgical instruments are used to complete the same steps as open surgery under video surveillance to achieve the same surgical results. In recent years, minimally invasive surgery, which has the advantages of small trauma, fast recovery, light pain and high cure rate, has developed rapidly. Laparoscopy, as a representative of minimally invasive surgery, has been widely used in the field of surgery, which involves many kinds of diseases and surgeries, and has been welcomed by the patients. Moreover, with the continuous progress of science and technology and the continuous improvement and innovation of surgical instruments, the room for laparoscopy will be more and more large.

Due to the limitations of space within the abdominal or thoracic cavity, minimally invasive surgical instruments are necessarily smaller and longer to allow for precise manipulation. The relatively difficult maneuver within minimally invasive laparoscopic surgery is undoubtedly suture. In the past, suturing of body organs or tissues through endoscopic surgery was achieved through the use of sharp metal suture needles, with a certain length of suture material tied to one end of said suture needle, and the surgeon operated the needles to pierce and pass through the body tissues, dragging the suture material through the body tissues, and once the suture material had been dragged through the body tissues the surgeon tied a knot in the suture material, with the knot in the suture material permitting the knot in the suture material allows the surgeon to adjust the tension of the suture material to fit the particular tissue being sutured and to control the approximation, closure, union, or other condition of the tissue. The ability to control tension is extremely important to the surgeon, independent of the type of surgical procedure being performed. In traditional suturing, the need to use both hands to coordinate the suture needle and the needle holding instrument to continuously suture in a limited space is found by many surgeons to be extremely difficult, and the exposure of the suture needle can lead to accidents, such as puncturing other organs of the patient or injuries to the surgeon or nurses.

With the development of technology, semi-manual suture device has been used in surgery, in the operation of semi-manual suture device for surgery, by holding the firing trigger, a cycle that the trigger is fully held down and fully open reset to achieve the needle into the suture tissue, and then hold the firing trigger to achieve complete penetration of the needle into the tissue, the needle is returned to the position, such as the return to the position, you need to hold the trigger several times repeatedly, so an operation requires the surgeon to Therefore, one operation requires the surgeon to hold the trigger manually several times in a row, which is laborious and requires a long suturing time.

### Contents of the invention

### (I) Technical problem

The present invention is to solve the technical problem of laborious operation of the suturing device in the prior art.

### (II) Technical solution

To solve the above technical problem, the present invention provides the following technical solution:

An electric suturing device, comprising: a working-end assembly, a main drive mechanism, a bending drive mechanism and a rotating drive mechanism; the working-end assembly comprises a suturing assembly and a bending connecting tube, wherein the main drive mechanism is used to drive a needle-and-thread component, to rotate in a reciprocal manner, to suture a to-be-sutured tissue, the bending drive mechanism is used to drive the working-end assembly to swing left and right, in order to adjust the position of the working-end assembly relative to the to-be-sutured tissue, and the rotating drive mechanism is used to drive the suturing assembly of the working-end assembly to achieve 360-degree rotation around the axis of the working end, so as to adjust the position of the needle-and-thread component relative to the entry point of the to-be-sutured tissue.

By setting the main drive mechanism, the bending drive mechanism and the rotating drive mechanism, the present invention can facilitate the doctor in adjusting the position of the suture component and performing the suture operation, so that the efficiency of suture can be improved and the operation time can be shortened.

Further, the electric suturing device further comprises a support assembly, the support assembly comprising: a main skeleton, a sub-skeleton, a right housing, a left housing, a handle; a power supply is detachably mounted in the handle, and the main skeleton and the sub-skeleton are used to fixedly mount the main drive mechanism, the bending drive mechanism and the rotating drive mechanism.

Further, the electric suturing device further comprises an extension tube, a distal end of the extension tube is connected to the working-end assembly, the extension tube is used to pass through a surgical passage to push the working-end assembly to a position to be sutured.

Further, the main drive mechanism comprises: a main drive motor, an eccentric wheel, a buffer, an extension rod, a flexible shaft; the main drive motor drives the eccentric wheel to rotate to drive the buffer to make a linear reciprocating motion, a distal end of the buffer is connected to a proximal end of the extension rod, a distal end of the extension rod is fixedly connected to a proximal end of the flexible shaft, and a distal end of the flexible shaft is connected to the working-end assembly.

Further, the working-end assembly comprises a suturing assembly and a bending connecting tube, wherein the suturing assembly comprises: a rack component, a gear component, a connecting rod component, a base; wherein the rack component and the gear component are engaged, and the flexible shaft is fixedly connected to an end of the rack component; the connecting rod component is provided with a pin hingedly matched with a waist-shaped hole provided in the gear component; the connecting rod component is provided with an ejector pin rod base, which is inserted into a curved groove of the base, thereby forming an inverse quadrilateral crank slider mechanism.

Further, the main drive mechanism, the bending drive mechanism and the rotating drive mechanism are able to be operated independently, and the operations of the three do not affect each other.

Further, the bending drive mechanism includes: a bending drive motor, a bending motor gear box component, a bending connecting rod, a bending pull piece and a bending joint; the bending motor gear box component includes a transmission screw and a connecting block, the bending drive motor drives the transmission screw to rotate, so as to drive a connecting block to reciprocate in linear motion, the connecting block is connected to the bending connecting rod, reciprocating linear motion of the connecting block drives the bending pull piece connected to the bending connecting rod to push and pull, thereby driving the working-end assembly to bend around two connection pins provided at the bending joint.

Further, the rotating drive mechanism includes a center gear, a D-shaped hole is provided in the center of the center gear, and the extension rod passes through the D-shaped hole in the center of the center gear;

the rotating drive mechanism further comprises an electric drive structure and/or a manually driving structure, wherein,

the electric drive structure comprises: a reduction motor, a motor gear; the motor gear engages with the center gear, the reduction motor rotates to drive the motor gear to rotate, the motor gear drives the center gear to rotate in the reverse direction, the center gear drives the extension rod to rotate in the reverse direction, and transmits the rotation through the flexible shaft to drive the suturing assembly of the working-end assembly to perform the rotational movement;

the manually driving structure comprises: a rotating head, an inert gear, a driving gear, the rotating head is provided with an internal gear, the internal gear, the inert gear, the driving gear, and the center gear engages with each other, and manually rotate the rotating head to drive the extension rod to rotate through the internal gear, the inert gear, the driving gear, the center gear, and transmit the rotation through the flexible shaft, thereby driving the suturing assembly of the working-end assembly to rotatably move.

Further, the electric suturing device further comprises a manipulation electric control assembly, the manipulation electric control assembly comprising a button module and a circuit module; wherein the button module comprises: a main button, a left button, a right button; wherein the main button is used to control the main drive mechanism and the bending drive mechanism, and the left button and the right button are used to control the rotating drive mechanism.

Further, the left button, the right button are symmetrically designed, so as to facilitate an operator to switch between left and right hands in operation.

With such a design, the invention has at least the following advantages:
(1) In the invention, through setting the main drive mechanism, the bending drive mechanism and the rotating drive mechanism, the doctor can facilitate in adjusting the position of the suture assembly and carrying out the suture operation, so that the efficiency of the suture can be improved and the operation time can be shortened.
(2) The electric suturing device of the present invention is easy to operate and can be operated with one hand, reducing the time-consuming of device adjustment operation and saving surgical time.
(3) The structure of the present invention is simple and effective, and the cost is low and easy for process realization.

### Description of the accompanying drawings

The above is only an overview of the technical solution of the present invention, in order to be able to more clearly understand the technical means of the present invention, the following combines the accompanying drawings with specific embodiments of the present invention for further detailed description.
FIG. 1 is a schematic diagram of the decomposition of components of the electric suturing device of the present invention;
FIG. 2 is a schematic diagram of the sectional structure of the components of the electric suturing device of the present invention;
FIG. 3 is a schematic diagram of the structure of the main drive mechanism of the present invention;
FIG. 4 is a schematic diagram of section of the working-end assembly of the present invention;
FIG. 5 is a schematic diagram of the internal structure of the working-end of the present invention;
FIG. 6 is a schematic diagram of the operation principle of the working-end assembly of the present invention;
FIG. 7 is a schematic diagram of the bending drive mechanism of the present invention;
FIG. 8 is a schematic diagram of the rotating drive mechanism of the present invention;
FIG. 9 is a schematic diagram of the sectional structure of the rotating drive mechanism of the present invention.

### [Illustration of accompanying markings]

1000 support assembly, 1100 main skeleton, 1200 sub-skeleton, 1300 right housing, 1400 left housing, 1401 handle, 1500 upper housing, 1600 extension tube, 2000 power supply, 3000 electric control assembly, 3100 button module, 3101 left button, 3102 right button, 3103 main button, 3200 circuit module, 4000 main drive mechanism, 4100 main drive motor, 4200 eccentric wheel, 4300 buffer, 4400 extension rod, 4500 flexible shaft, 5000 bending drive mechanism, 5100 bending motor gear box component, 5101 transmission screw, 5102 connecting block, 5200 bending connecting rod, 5300 bending pull piece, 5400 bending joint, 5500 connecting pins, 6000 rotating drive mechanism, 6100 gear box component, 6101 center gear, 6102 motor gear, 6103 inert gear, 6104 drive gear, 6200 reduction motor, 6300 rotating head, 6301 internal gear, 7000 working-end assembly, 7100 rack component, 7200 gear component, 7201 waist-shaped hole, 7300 connecting rod component, 7301 ejector rod base, 7302 pin, 7400 base, 7401 curved groove, 7500 bending connecting tube, and 7600 needle-and-thread component.

### Specific embodiments of the invention

The technical solutions in the embodiments of the present invention will be clearly and completely described below in conjunction with the accompanying drawings in the embodiments of the present invention, and it is clear that the described embodiments are only a part of the embodiments of the present invention and not all of the embodiments. Based on the embodiments in the present invention, all other embodiments obtained by persons of ordinary skill in the art without making creative labor are within the scope of protection of the present invention.

In this document, "up", "down" and the like are used only to indicate relative positional relationships between related parts, and not to define the absolute positions of these related parts.

Each of the following parts is defined as a proximal end near the operator and a distal end away from the operator. This expression proximal end and distal end is used only to make the description simple and clear and should not be construed as any limitation of the present invention.

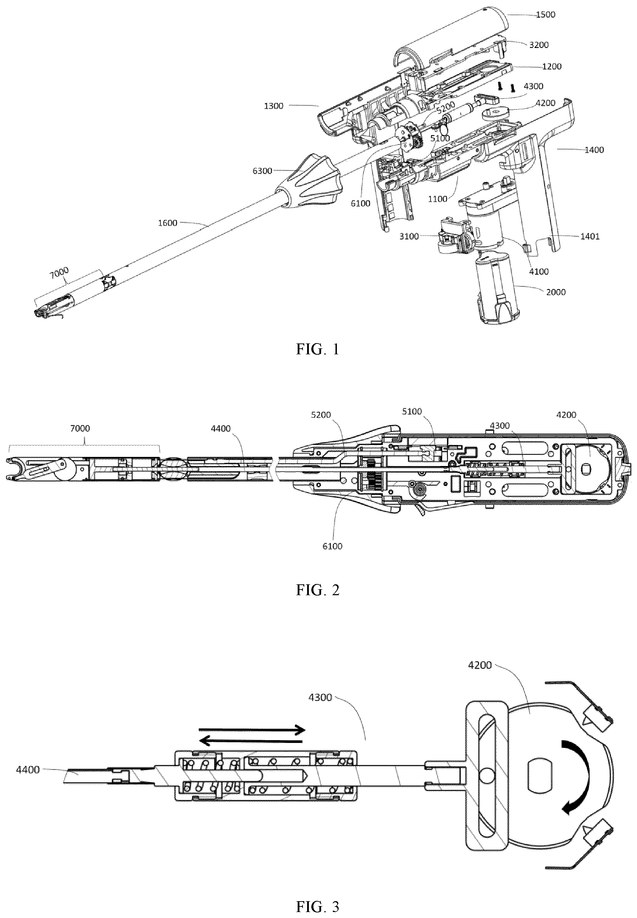

Referring to FIGS. 1-5, an electric suturing device comprising: a working-end assembly 7000, a main drive mechanism 4000, a bending drive mechanism 5000 and a rotating drive mechanism 6000; the working-end assembly 7000 comprises a suture component and a bending connecting tube, wherein the main drive mechanism 4000 is used to drive a needle-and-thread component 7600, to rotate in a reciprocal manner, to suture a to-be-sutured tissue, the bending drive mechanism 5000 is used to drive the working-end assembly 7000 to swing left and right to adjust the position of the working-end assembly 7000 relative to the to-be-sutured tissue, and the rotating drive mechanism 6000 is used to drive the suture assembly to achieve a 360-degree rotation around the axis of the suture assembly so as to adjust the position of the needle-and-thread component 7600 relative to the entry point of the to-be-sutured tissue.

The electric suturing device further comprises a support assembly 1000, the support assembly comprising: a main skeleton 1100, a sub-skeleton 1200, a right housing 1300, a left housing 1400, a handle 1401; a power supply 2000 is detachably mounted within the handle, the main skeleton and the sub-skeleton are used to fixedly mount the main drive mechanism 4000, the bending drive mechanism 5000 and the rotating drive mechanism 6000. The power supply 2000 provides power supply drive for the main drive mechanism 4000, the bending drive mechanism 5000 and the rotating drive mechanism 6000, and the power supply can be quickly plugged in to the handle 1401, and the main skeleton 1100 and the sub-skeleton 1200 are used as the main support members for installing the three drive mechanisms, and are used for realizing fixed installation of the three drive mechanisms.

The electric suturing device is also provided with an extension tube 1600, a distal end of the extension tube 1600 is connected to the working-end assembly 7000, and the extension tube is used to pass through the surgical passageway to push the working-end assembly 7000 to the position to be sutured.

The main drive mechanism 4000 includes: a main drive motor 4100, an eccentric wheel 4200, a buffer 4300, an extension rod 4400 and a flexible shaft 4500. The main drive motor 4100 drives the eccentric wheel 4200 to rotate to drive the buffer 4300 to do a straight line reciprocating motion, the distal end of the buffer 4300 is connected to the extension rod 4400, the distal end of the extension rod 4400 is fixedly connected to the flexible shaft 4500, the main drive mechanism 4000 drives the working-end assembly 7000 through the flexible shaft 4500 to perform a suturing operation. The distal end of the flexible shaft 4500 is fixedly connected to the working-end assembly 7000; the main drive mechanism 4000 drives the working-end assembly 7000 through the flexible shaft 4500 to carry out the suturing operation. Wherein, the buffer component includes: a buffer spring, a buffer rod, a buffer sleeve; the buffer component is capable of realizing buffering in the pulling and pushing process. Preferably, the extension rod is secured to the flexible shaft in a manner that includes, but is not limited to, welding, interference press fitting, and the like. The flexible shaft 4500 is a shaft with little rigidity and elasticity for free bending and transmission, which is used for coupling two shafts that are not in the same axis and not in the same direction or have relative motions in order to transmit rotary motions and torque, and is able to transmit rotary motions and torque flexibly to any position.

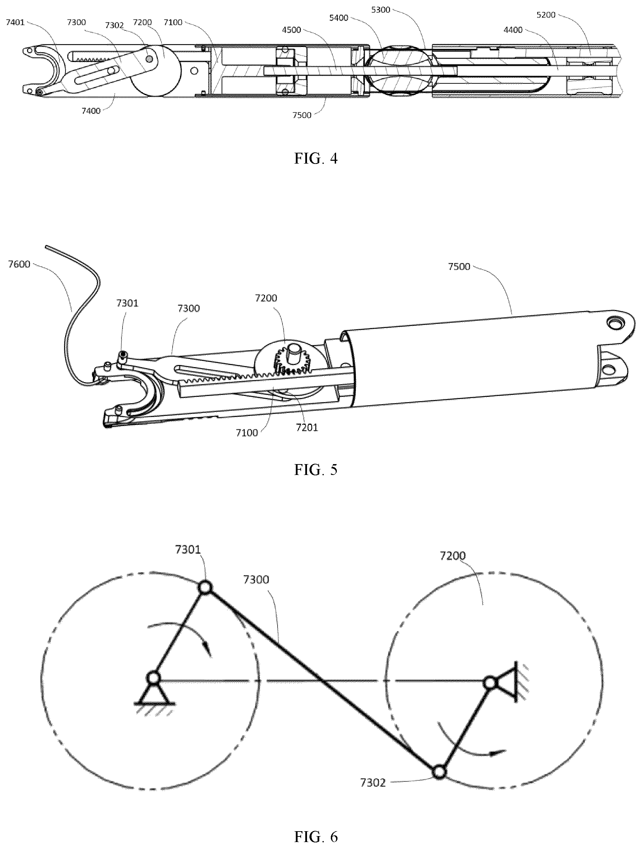

Referring to FIGS. 5-6, the working-end assembly 7000 comprises a suturing assembly and a bending connecting tube 7500, wherein the suturing assembly comprises: a rack component 7100, a gear component 7200, a connecting rod component 7300, and a base 7400. Wherein, the rack component 7100 is engaged with the gear component 7200, and the flexible shaft 4500 is fixedly connected to one end of the rack component 7100. The connecting rod component 7300 is provided with a pin 7302 hingedly matched with a waist-shaped hole 7201 provided in the gear component 7200; the connecting rod component 7300 is provided with an ejector rod base 7301 fitted into an curved groove 7401 of the base 7400 so as to form an inverse-quadrilateral crank-slider mechanism. Pushing and pulling the rack member 7100 back and forth through the flexible shaft 4500 can link the gear component 7200 to rotate, and the rotation of the gear member 7200 drives the connecting rod member 7300 to swing, drives the ejector rod base 7301 to swing back and forth in the curved groove 7401, so as to realize the suturing operation.

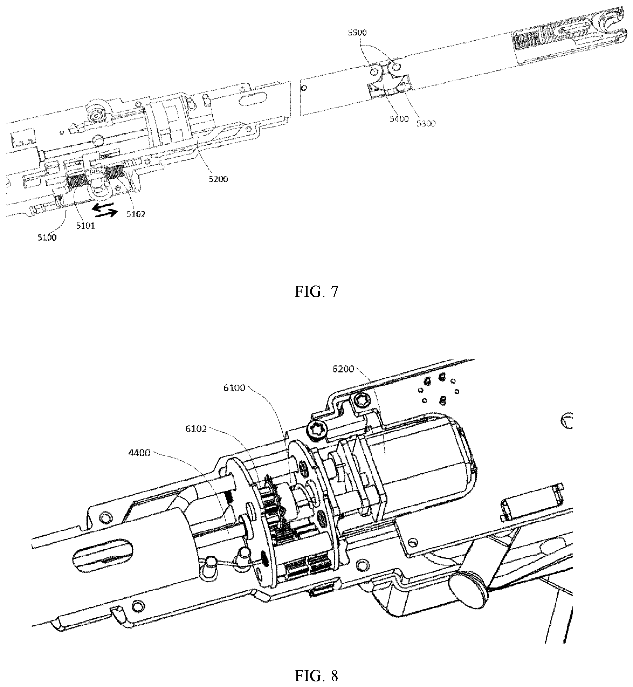

The main drive mechanism 4000, the bending drive mechanism 5000 and the rotating drive mechanism 6000 can be operated independently, and the operations of the three do not affect each other. Referring to FIG. 7, the bending drive mechanism 5000 includes: a bending drive motor, a bending motor gear box part 5100, a bending connecting rod 5200, a bending pull piece 5300 and a bending joint 5400. The bending drive motor drives the bending motor gear box part 5100 to output power, the bending motor gear box part 5100 includes a transmission screw 5101 and a connecting block 5102, the bending drive motor drives the transmission screw 5101 to rotate to drive the connecting block 5102 to reciprocate linearly, the connecting block 5102 is connected to the bending connecting rod 5200, and the reciprocating motion of the connecting block 5102 drives the bending pull piece 5300 connected to the bending connecting rod 5200 to push and pull the motion, thereby driving the working-end assembly 7000 to bend around the two connecting pins 5500 provided at the bending joint 5400. The flexible shaft 4500 passes through the bending joint 5400, and when the working-end assembly bends, the centrally located flexible shaft 4500 also bends with the working-end assembly, and the flexible shaft 4500, due to its intrinsic properties, can also transmit push-pull and torsion forces after bending.

Referring to FIGS. 8-9, the rotating drive mechanism includes a center gear 6101, a D-shaped hole is provided in the center of the center gear 3101, and an extension rod passes through the D-shaped hole in the center of the center gear, and the rotating drive mechanism 6000 includes an electric and/or a manual drive structure, wherein the electric drive structure comprises: a reduction motor 6200, a center gear 6101, the center gear 6101 and a motor gear 6102 constitute a gear box component 6100; a bend connecting tube 7500 of the working-end assembly is hingedly connected to the bending joint. The motor gear engages with the center gear, the reduction motor 6200 rotates to drive the motor gear to rotate, and the motor gear rotates to drive the center gear 6101 to rotate in the reverse direction, thereby driving the extension rod 4400 to rotate with the reverse direction, and transmitting the rotation through the flexible shaft 4500 to thereby drive the suturing assembly of the working-end component 7000 to rotatably move. By matching the extension rod 4400, which has a D-shaped structure in cross-section, with the D-shaped hole provided in the center of the gear box component 6100, so as to make the linear reciprocating motion of the extension rod driven by the main drive mechanism and the rotary motion of the extension rod driven by the rotating drive mechanism not affect each other.

The manual driving structure includes: a rotary head 6300, an inert gear 6103, a drive gear 6104, the rotary head 6300 is provided with an inner gear 6301, the inner gear 6301, the inert gear 6103, the drive gear 6104 and the center gear 6101 engage with each other, and manually rotating the rotary head 6300 can drive the extension rod through the inner gear 6301, the inert gear 6103, the drive gear 6104 and center gear 6101 to rotate and transmit rotation through the flexible shaft 4500, thereby driving the suturing assembly of the working-end assembly 7000 to rotatably move. When both manual and electric drive structures are provided, switching arbitrarily between electric rotation and manual rotation operation can be realized.

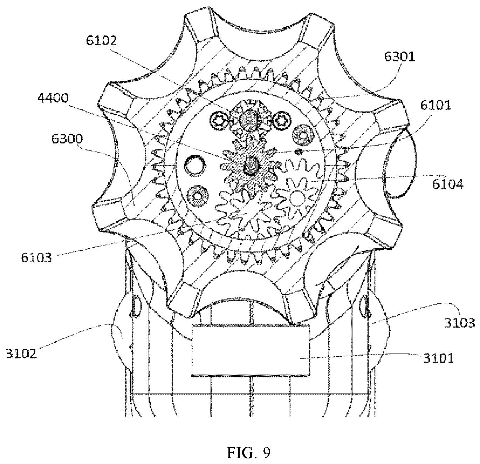

Referring to FIGS. 1 and 9, further, the electric suturing device also includes a manipulation electric control assembly 3000, the manipulation electric control assembly 3000 includes a button module 3100 and a circuit module 3200. The button module 3100 includes: a main button 3103, a left button 3101 and a right button 3102. Wherein, the main button is used to control the main drive mechanism and the bending drive mechanism, and the left button and the right button are used to control the rotating drive mechanism. Wherein, the left button 3101 and the right button 3102 are symmetrically designed to facilitate the operator's left hand and right hand to commutatively operate the device. The circuit module 3200 is pre-programmed for controlling the overall electric control system, which includes motor driving, voltage detecting, current detecting and status indication, etc.

Other embodiments of the present invention will readily come to mind by those skilled in the art upon consideration of the invention disclosed in the specification and embodiments. This application is intended to cover any variations, uses, or adaptations of the present invention that follow the general principles of the invention and include common knowledge or customary technical means in the art not disclosed herein. The specification and embodiments are to be regarded as exemplary only, and the true scope and spirit of the invention is indicated by the following claims.

It should be understood that the present invention is not limited to the precise structure which has been described above and illustrated in the accompanying drawings, and that various modifications and alterations may be made without departing from its scope. The scope of the invention is limited only by the appended claims.

## Claims

1. An electric suturing device, comprising: a working-end assembly, a main drive mechanism, a bending drive mechanism and a rotating drive mechanism; the working-end assembly comprises a suturing assembly and a bending connecting tube, wherein the main drive mechanism is used to drive a needle-and-thread component, to rotate in a reciprocal manner, so as to suture a to-be-sutured tissue, the bending drive mechanism is used to drive the working-end assembly to swing left and right, in order to adjust the position of the working-end assembly relative to the to-be-sutured tissue, and the rotating drive mechanism is used to drive the suturing assembly of the working-end assembly to achieve 360-degree rotation around the axis of the working end, so as to adjust the position of the needle-and-thread component relative to the entry point of the to-be-sutured tissue.

2. The electric suturing device as claimed in claim 1, **characterized in that** the electric suturing device further comprises a support assembly, the support assembly comprising: a main skeleton, a sub-skeleton, a right housing, a left housing and a handle; a power supply is detachably mounted in the handle, and the main skeleton and the sub-skeleton are used to fixedly mount the main drive mechanism, the bending drive mechanism and the rotating drive mechanism.

3. The electric suturing device as claimed in claim 1, **characterized in that** the electric suturing device further comprises an extension tube, a distal end of the extension tube is connected to the working-end assembly, the extension tube is used to pass through a surgical passage to push the working-end assembly to a position to be sutured.

4. The electric suturing device as claimed in claim 1, **characterized in that** the main drive mechanism comprises: a main drive motor, an eccentric wheel, a buffer, an extension rod, and a flexible shaft; the main drive motor drives the eccentric wheel to rotate to drive the buffer to make a linear reciprocating motion, a distal end of the buffer is connected to a proximal end of the extension rod, a distal end of the extension rod is fixedly connected to a proximal end of the flexible shaft, and a distal end of the flexible shaft is connected to the working-end assembly.

5. The electric suturing device as claimed in claim 4, **characterized in that** the working-end assembly comprises a suturing assembly and a bending connecting tube, wherein the suturing assembly comprises: a rack component, a gear component, a connecting rod component, a base; wherein the rack component and the gear component are engaged, and the flexible shaft is fixedly connected to an end of the rack component; the connecting rod component is provided with a pin hingedly matched with a waist-shaped hole provided in the gear component; the connecting rod component is provided with an ejector pin rod base, which is inserted into a curved groove of the base, thereby forming an inverse quadrilateral crank slider mechanism.

6. The electric suturing device as claimed in claim 1, **characterized in that** the main drive mechanism, the bending drive mechanism and the rotating drive mechanism are able to be operated independently, and the operations of the three do not affect each other.

7. The electric suturing device as claimed in any one of claims 1-6, **characterized in that** the bending drive mechanism includes: a bending drive motor, a bending motor gear box component, a bending connecting rod, a bending pull piece and a bending joint; the bending motor gear box component includes a transmission screw and a connecting block, the bending drive motor drives the transmission screw to rotate, so as to drive a connecting block to reciprocate in linear motion, the connecting block is connected to the bending connecting rod, reciprocating linear motion of the connecting block drives the bending pull piece connected to the bending connecting rod to push and pull, thereby driving the working-end assembly to bend around two connection pins provided at the bending joint.

8. The electric suturing device as claimed in any one of claims 4-6, **characterized in that** the rotating drive mechanism includes a center gear, a D-shaped hole is provided in the center of the center gear, and the extension rod passes through the D-shaped hole in the center of the center gear;
the rotating drive mechanism further comprises an electric drive structure and/or a manually driving structure, wherein,
the electric drive structure comprises: a reduction motor, a motor gear; the motor gear engages with the center gear, the reduction motor rotates to drive the motor gear to rotate, the motor gear drives the center gear to rotate in the reverse direction, the center gear drives the extension rod to rotate in the reverse direction, and transmits the rotation through the flexible shaft to drive the suturing assembly of the working-end assembly to perform the rotational movement;
the manually driving structure comprises: a rotating head, an inert gear, a driving gear, the rotating head is provided with an internal gear, the internal gear, the inert gear, the driving gear, and the center gear engages with each other, and manually rotate the rotating head to drive the extension rod to rotate through the internal gear, the inert gear, the driving gear, the center gear, and transmit the rotation through the flexible shaft, thereby driving the suturing assembly of the working-end assembly to rotatably move.

9. The electric suturing device as claimed in any one of claims 1-6, **characterized in that** said electric suturing device further comprises a manipulation electric control assembly, the manipulation electric control assembly comprising a button module and a circuit module; wherein the button module comprises: a main button, a left button, a right button; wherein the main button is used to control the main drive mechanism and the bending drive mechanism, and the left button and the right button are used to control the rotating drive mechanism.

10. The electric suturing device as claimed in claim 9, **characterized in that** the left button and the right button are symmetrically designed, so as to facilitate an operator to switch between left and right hands in operation.
